# EUROPEAN PATENT SPECIFICATION

(11) **EP 3 021 903 B1**
(45) Date of publication and mention of the grant of the patent: **30.08.2017**
(21) Application number: 14741612.7
(22) Date of filing: 18.07.2014
(51) Int. Cl.: A61M 5/32, A61M 5/34, A61M 5/31

(54) **A TIP CAP AND AN INJECTION DEVICE HAVING A DISTAL TIP SEALED BY A TIP CAP**
SPITZENKAPPE UND INJEKTIONSVORRICHTUNG MIT DISTALER, DURCH EINE SPITZENKAPPE ABGEDICHTETER SPITZE
CAPUCHON DE POINTE ET DISPOSITIF D'INJECTION PRÉSENTANT UNE POINTE DISTALE SCELLÉE PAR UN CAPUCHON DE POINTE

(30) Priority: 18.07.2013 EP 13306033
(43) Date of publication of application: 25.05.2016
(73) Proprietor: Becton Dickinson France, 38800 Le Pont-de-Claix (FR)
(72) Inventor: DELEUIL, Nicolas, F-38000 Grenoble (FR); BENSALLAH, Moussa, 38800 Pont-de-Claix (FR)
(74) Representative: Delorme, Nicolas
(86) International application number: PCT/EP2014/065548
(87) International publication number: WO 2015/007900

(56) References cited:
- EP-A1- 2 253 349
- EP-A1- 2 266 646
- WO-A2-2010/091522
- US-A1- 2008 281 264
- US-A1- 2009 287 160

## Description

The present invention relates to a tip cap designed to be sealingly mounted on a distally projecting tip of an injection device. The invention also relates to a tip cap assembly comprising such a tip cap, and to an injection device equipped with such a tip cap or tip cap assembly.

In this application, the distal end of a component or apparatus must be understood as meaning the end furthest from the hand of the user and the proximal end must be understood as meaning the end closest to the hand of the user, with reference to the injection device intended to be used with said component or apparatus. As such, in this application, the distal direction must be understood as the direction of injection with reference to the injection device, and the proximal direction is the opposite direction, i.e. for example the direction of a transfer of the product from a medical container to the injection device.

Usually, a conventional injection device such as a syringe includes a barrel having an open proximal end and a distal end, as well as a cylindrical wall extending between both ends and defining a retaining chamber designed to contain a medical fluid to be either expelled or withdrawn. An elongate tip projects from the distal end of the barrel and includes a narrow passage which communicates with the substance retaining chamber of the barrel.

In some applications, the medical fluid is pre-filled into the injection device barrel and can be stored for a considerable period of time before use. Therefore, the barrel must be sealed to prevent contamination or loss of the medication. Seals also prevent health care workers from being needlessly exposed to medications.

To that end, a tip cap is usually provided. Such a tip cap is sealingly mounted on the injection device tip in order to prevent leakage and to avoid any contamination of the medication. A conventional tip cap is frictionally engaged with the injection device tip, and may be removed from the injection device tip shortly prior to the use of the injection device. US2009/0287160 discloses such a tip cap. The hub of a needle assembly or a connector may then be securely engaged with the injection device tip, and the medical fluid can be urged through the passage in the tip by a distal sliding movement of a plunger rod inside the barrel.

Elastomeric tip caps from the prior art, when plugged on the distal end of pre-filled syringe tips, generally perform well, but in some cases, they may not provide the required complete sealing level.

This could happen for example when the manufacturing equipment used for plugging the tip cap on the injection device tip does not provide an appropriate positioning, for example when the tip cap is inserted either too far or not far enough with respect to the most distal part of the injection device tip.

Besides, the tip cap may be accidentally moved relative to the injection device tip due to inadvertent forces imposed thereon. Moreover, dimensional changes or instability of the elastomeric seal may lead to a change in the positioning of the tip cap relative to the injection device tip, resulting in a less efficient cooperation between the tip cap and the injection device tip, and ultimately to a risk of leakage.

Therefore, it would be desirable to provide a tip cap that would ensure a very good sealing with the injection device tip, over the whole storage period of the pre-filled injection device.

According to a first aspect, the invention relates to a tip cap designed to be sealingly mounted on a distally projecting tip of an injection device having a passageway extending therethrough, the tip cap having an axis and comprising:
- a closed distal end portion having a substantially transverse proximal surface;
- a substantially cylindrical peripheral skirt extending in the proximal direction from the proximal surface of the distal end portion, said peripheral skirt having an inner surface designed to cooperate with the outer surface of the injection device tip;
- a nipple protruding from the proximal surface of the distal end portion in the proximal direction, said nipple having a dome-shaped proximal portion and being designed to engage the injection device tip;
an inner cavity thus being formed in the tip cap, the bottom of said cavity being a part of the proximal surface of the distal end portion and having a substantially annular shape which is connected to the peripheral skirt inner surface by an outer fillet and to the nipple by an inner fillet.

According to the invention, the tip cap further has the following features:
- the outer fillet has a radius of curvature ranging from 0,4 to 0,6 mm;
- the inner fillet has a radius of curvature ranging from 0,25 to 0,35 mm;
- the nipple base width, substantially in the plane of the proximal surface of the distal end portion, ranges from 1,4 to 1,6 mm;
- the dome-shaped proximal portion of the nipple has a radius of curvature ranging from 0,55 to 0,70 mm.

In this application, the direction of the tip cap axis is the longitudinal direction, while the terms "radial" and "transverse" refer to a direction orthogonal to the longitudinal direction. The term "outer" refer to elements located further from the longitudinal axis than "inner" elements, in the radial direction.

The term "fillet" means the curved and concave junction area between two surfaces.

The "radius of curvature" means the radius of the arc of a circle which comes closest to the rounded form of the component under consideration.

It has been found that, thanks to the above combination of features, the sealing cooperation between the tip cap and the injection device tip is particularly good. Therefore, this combination of features greatly improves the sealing efficiency of the tip cap with respect to prior art tip caps, not only when the tip cap is adequately positioned onto the injection device tip, but also when the positioning is not fully adequate, either initially or during the storage period of the pre-filled injection device, for the above mentioned reasons.

Moreover, with this invention, the increase in the sealing performances of the tip cap does not have any impact on the other usual tip cap performances. In particular, the pull out force, i.e. the force required to remove the tip cap from the injection device tip prior to the use of the injection device, is not increased. In addition, the sealing properties of the tip cap according to the invention remains stable even after a conventional sterilization process, typically with ethylene oxide, steam or gamma irradiation.

Another significant advantage of the invention is that, because of the optimized features of the tip cap, there are fewer constraints in the choice of the physical properties of the tip cap material for efficiently preventing leakage. More particularly, thanks to the specific features of the tip cap design, whatever the intrinsic properties of the elastomeric material, the tip cap will remain hermetic, ensuring a good storage of the medical fluid contained in the injection device.

According to a non limitative embodiment of the invention, the tip cap may further be such that:
- the outer fillet has a radius of curvature ranging from 0,45 to 0,55 mm, for example around 0,5 mm;
- and/or the inner fillet has a radius of curvature ranging from 0,27 to 0,33 mm, for example around 0,3 mm;
- and/or the nipple base width, substantially in the plane of the proximal surface of the distal end portion, ranges from 1,45 to 1,55 mm, and is for example around 1,5 mm;
- and/or the dome-shaped proximal portion of the nipple has a radius of curvature ranging from 0,6 to 0,70 mm, for example around 0,65 mm.

Besides, the nipple axial length can range from 1,2 to 1,4 mm, and can be for example around 1,3 mm.

The tip cap may preferably be made of an elastomeric material. Suitable materials for the tip cap 1 of the invention include natural rubber, acrylate-butadiene rubber, cis-polybutadiene, chloro or bromobutyl rubber, chlorinated polyethylene elastomers, polyalkylene oxide polymers, ethylene vinyl acetate, fluorosilicone rubbers, hexafluoropropylene-vinylidene fluoride-tetrafluoroethyleneterpolymers, butyl rubbers, polyisobutene, synthetic polyisoprene rubber, silicone rubbers, styrene-butadiene rubbers, tetrafluoroethylene propylene copolymers, thermoplastic-copolyesters, thermoplastic elastomers, or the like or a combination thereof.

The tip cap can be the only element plugged on the injection device tip, or can be part of a tip cap assembly.

Thus, according to a second aspect, the invention relates to a tip cap assembly for use with an injection device having a passageway extending therethrough and having a distally projecting tip, the tip cap assembly comprising:
- a collar securely engageable around the injection device tip;
- a tip cap as previously described, sealingly engageable with the injection device tip;
- a rigid outer cap securely disposed around the tip cap, and having a proximal portion engageable with the collar, said rigid outer cap comprising tamper indicator means for indicating a separation of the rigid outer cap and the collar.

Alternatively, the tip cap assembly can comprise only a rigid outer cap mounted on the tip cap plugged on the injection device, to allow a better gripping of the tip cap when one needs to remove it, and/or to ensure that no one can prick with a needle, when such a needle is present on the injection device tip.

According to a third aspect, the invention relates to an injection device comprising:
- a barrel having a passageway extending therethrough and having a distally projecting tip;
- and a tip cap as previously described, the tip cap being sealingly mounted on the injection device tip; or a tip cap assembly as previously described, the tip cap assembly being mounted on the injection device tip so that the tip cap sealingly engages the injection device tip.

These and other features and advantages will become apparent upon reading the following description in view of the drawings attached hereto representing, as non-limiting examples, embodiments of the invention.
Figure 1 is a perspective view of a tip cap according to an embodiment of the invention;
Figure 2 is a longitudinal cross section of the tip cap of Figure 1 mounted on a tip of an injection device tip (the injection device being partially shown);
Figure 3 is a distal view of the tip cap of Figure 1;
Figures 4 and 5 are cross-sections of the tip cap, respectively along lines IV-IV and V-V of Figure 3;
Figure 6 is a detailed view of the bottom of the inner cavity of the tip cap;
Figure 7 is a perspective view of an embodiment of an injection device equipped with a needle assembly, after removal of the tip cap;
Figure 8 is a longitudinal cross section and exploded view of another embodiment of an injection device equipped with a needle assembly, after removal of the tip cap;
Figure 9 is a longitudinal cross section and exploded view of an embodiment of an injection device equipped with a tip cap assembly;
Figure 10 is a longitudinal cross section of an injection device equipped with a tip cap according to an embodiment of the invention, for illustrating the calculation method of the tip cap insertion depth used for leak tests conducted on such a tip cap;
Figures 11 to 13 are graphs showing the results of a first leak test, respectively for a tip cap of the prior art, for a tip cap according to a first embodiment of the invention, and for a tip cap according to a second embodiment of the invention;
Figures 14 to 16 are graphs showing the results of a second leak test, respectively for a tip cap of the prior art, for a tip cap according to a first embodiment of the invention, and for a tip cap according to a second embodiment of the invention;
Figures 17 to 19 are graphs showing the results of a third leak test, respectively for a tip cap of the prior art, for a tip cap according to a first embodiment of the invention, and for a tip cap according to a second embodiment of the invention.

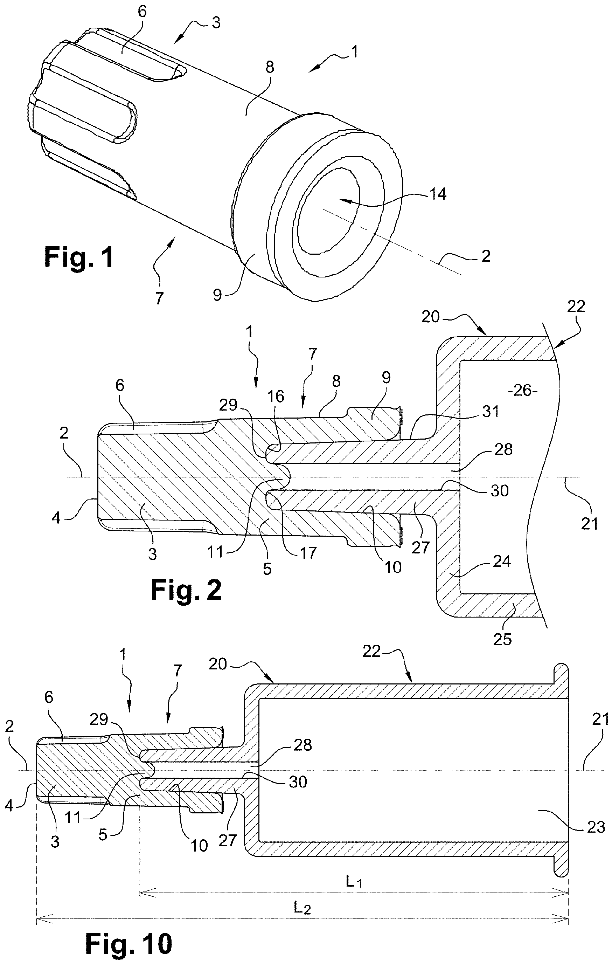

A tip cap 1 according to an embodiment of the invention is shown in Figures 1 to 6.

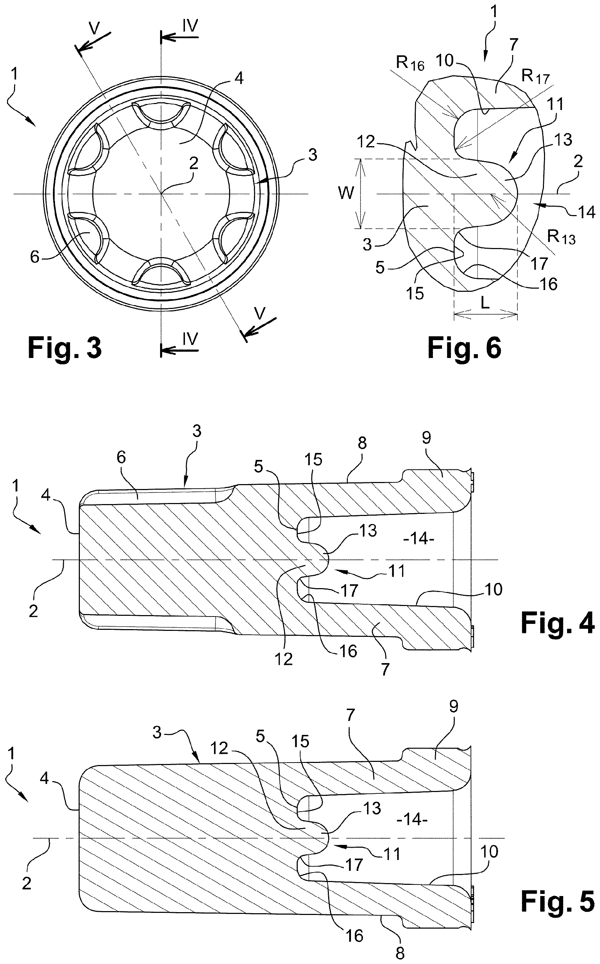

The tip cap 1 has a longitudinal axis 2. It comprises a closed distal end portion 3 which has a substantially transverse distal surface 4 and a substantially transverse proximal surface 5. The length of the closed distal end portion 3, along the longitudinal axis 2, can be around half the length of the tip cap 1. The closed distal end portion 3 can further comprise notches 6 extending longitudinally from the distal surface 4. The notches 6 are preferably substantially regularly spaced around the tip cap periphery, as shown in Figure 3. For example, the tip cap 1 can have six notches 6. Such notches 6 create a ribbed distal outer surface which helps to handle the tip cap 1.

The tip cap 1 also comprises a substantially cylindrical peripheral skirt 7 which extends in the proximal direction from the proximal surface 5 of the distal end portion 3. The skirt 7 has an outer surface 8 which is substantially cylindrical and level with the outer surface of the distal end portion 3, except at the proximal end where the tip cap 1 has an annular bead 9. The outer surface 8 of the tip cap 1 can be slightly conical and converging towards the distal surface 4. The skirt 7 also has an inner surface 10.

The tip cap 1 further comprises a nipple 11 which protrudes from the proximal surface 5 of the distal end portion 3, in the proximal direction. The nipple 11 has a distal portion 12 which is substantially conical and is converging in the proximal direction, as well as a proximal portion 13 which is substantially dome-shaped. The nipple 11 is substantially centrally located in the proximal surface 5.

Thus, an inner cavity 14 is formed inside the tip cap 1, said inner cavity being open at its proximal end and closed at its distal portion, by the bottom 15. The bottom 15 of the inner cavity 14 is a part of the proximal surface 5 of the distal end portion 3 and has a substantially annular shape. The bottom 15 is connected to the peripheral skirt inner surface 10 by an outer fillet 16 and to the nipple 11 by an inner fillet 17.

As shown in Figure 2, the tip cap 1 is designed to sealingly close a pre-filled injection device prior to its use.

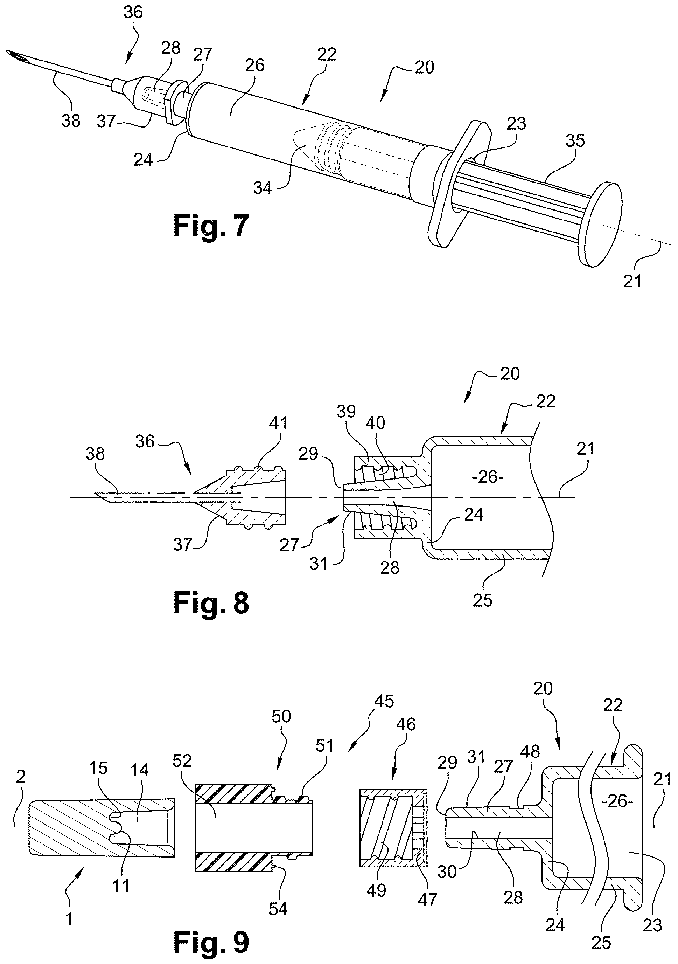

Such an injection device 20 has a longitudinal axis 21. It comprises a barrel 22 having an open proximal end 23 (see Figures 7 or 9) and a distal end 24, as well as a cylindrical wall 25 extending between the ends 23, 24 and defining a chamber 26 for retaining a medical fluid. An elongate tip 27 projects distally from the distal end 24 of the barrel 22 and includes a narrow passage 28 which communicates with the chamber 26 of the barrel. The injection device 20 therefore has a passageway extending therethrough, from its proximal end 23 towards the distal end 29 of the tip 27.

The barrel 22 can be made of plastic or glass. More preferably, the tip 27 can also be made of glass material and can have a ceramic coating to enhance the pull out force of the tip cap 1. Indeed, the roughness of the ceramic material allows a better adherence of the rubber tip cap 1 on the glass tip and avoids the sticking of the rubber on the glass, therefore leading to an easy removal of the tip cap from the tip.

The tip 27 has an inner surface 30 which can be substantially cylindrical, and an outer surface 31 which is preferably tapered in the distal direction by an angle of around 3,5°, to allow a Luer type connection, meaning a connection between the tip and for example a needle hub or a connector. The distal end 29 of the tip 27 is preferably substantially transverse.

When the tip cap 1 is adequately mounted on the injection device tip 27, in order to sealingly close the injection device 20 and prevent the substance contained in the chamber 26 from leaking, the surfaces of the inner cavity 14 of the tip cap 1 sealingly cooperate with the surfaces of the injection device tip 27.

More precisely, as shown in Figure 2:
- the inner surface 10 of the peripheral skirt 7 of the tip cap 1 cooperates with the outer surface 31 of the injection device tip 27. Said inner surface 10 therefore preferably has a tapered shape fitting to that of the outer surface 31 of the injection device tip 27;
- the nipple 11 is engaged inside the distal end portion of the tip 27;
- and, advantageously, the bottom 15 of the inner cavity 14 can furthermore cooperate with the distal end 29 of the tip 27.

The tip cap 1, which can preferably be made of an elastomeric material, is frictionally retained in engagement with the tip 27, thereby providing a sealed closure. In order to optimize the sealing effect, in various conditions and with different formulations of rubber, the tip cap 1 according to the invention has the following characteristics (see Figure 6):
- the outer fillet 16 has a radius of curvature R16 ranging from 0,4 to 0,6 mm;
- the inner fillet 17 has a radius of curvature R17 ranging from 0,25 to 0,35 mm;
- the nipple base width W, substantially in the plane of the proximal surface 5 of the distal end portion 3, i.e. in the plane of the bottom 15 of the inner cavity 14, ranges from 1,4 to 1,6 mm;
- and the dome-shaped proximal portion 13 of the nipple 11 has a radius of curvature R13 ranging from 0,55 to 0,70 mm.

According to possible embodiments of the invention, the tip cap 1 can have one or more of the following geometrical features:
- the radius of curvature R16 of the outer fillet 16 ranges from 0,45 to 0,55 mm, and is for example around 0,5 mm;
- the radius of curvature R17 of the inner fillet 17 ranges from 0,27 to 0,33 mm, and is for example around 0,3 mm;
- the nipple base width W ranges from 1,45 to 1,55 mm, and is for example around 1,5 mm;
- the radius of curvature R13 of the dome-shaped proximal portion 13 of the nipple 11 ranges from 0,6 to 0,70 mm, and is for example around 0,65 mm.

Another characteristic of the tip cap 1 which contributes to improving the sealing effect is the axial length L of the nipple 11 (see Figure 6). According to an embodiment of the invention, said axial length L can range from 1,2 to 1,4 mm, and it can be for example around 1,3 mm.

Two examples of a tip cap 1 according to the invention, and providing a very good sealing effect, are described in the following table.

| feature | tip cap 1 according to a first embodiment | tip cap 1 according to a second embodiment |
|---|---|---|
| R16 | 0,5 | 0,5 |
| R17 | 0,3 | 0,3 |
| W | 1,6 | 1,4 |
| R13 | 0,65 | 0,65 |
| L | 1,3 | 1,3 |

In practice, the injection device 20 can further comprise a plunger rod 35, which is inserted into the open proximal end 23 of the barrel 22 and is mounted on a stopper 34 capable of sliding inside the barrel 22 while maintaining a fluid-tight engagement with the cylindrical wall 25 of the barrel 22. After removal of the tip cap 1, a distal sliding movement of the plunger rod 35 urges the medical fluid out of the chamber 26 through the passage 28 in the tip 27.

After the tip cap 1 has been removed, a needle assembly 36 can be mounted on the injection device 20. Such a needle assembly 36 comprises a needle hub 37 which holds a needle 38 and can be engageable with mounting means on the tip 27.

The injection device 20 can be of various types, as depicted in Figures 7 to 9.

In an embodiment depicted in Figure 7, the mounting means on the barrel 22 only include the tip 27, to provide a Luer type connection with an appropriate needle hub 28. Alternatively, an additional rigid cap (not shown) can be provided around the tip cap 1 to protect the needle 38.

In another embodiment depicted in Figure 8, the mounting means on the barrel 22 further include an outer wall 39 projecting from the distal end 24 of the barrel 22 in the distal direction, disposed in spaced concentric relationship around the tip 27. The outer wall 39 has inner threads 40 for engagement with corresponding outer threads 41 arranged on a corresponding needle hub 37, for a Luer lock type connection of the needle assembly 36 on the injection device 20. Alternatively, another connector type such as an intravenous line (not shown) can be connected on the outer wall 39.

In still another embodiment depicted in Figure 9, the tip cap 1 is part of a tip cap assembly 45.

This tip cap assembly 45 comprises a collar 46 which is securely engageable around the injection device tip 27. This collar 46, which has an inner thread 49, forms a separate Luer lock collar and can be snap fitted onto the injection device tip 27, through the engagement of an inner ring 47 of the collar 46 preferably in an annular groove 48 arranged on the tip 27.

The tip cap assembly 45 further comprises a rigid outer cap 50. The rigid outer cap 50 has a through hole 52 for securely receiving the tip cap 1 according to the invention, and an outer thread 51 located on its proximal portion and designed to cooperate with the inner thread 49 of the collar 46. In the embodiment illustrated in Figure 9, the outer surface 8 of the skirt 7 of the tip cap 1 is substantially cylindrical and devoid of annular bead 9, the through hole 52 having a substantially cylindrical peripheral surface. Alternatively, the tip cap 1 could comprise the above described annular bead 9, the through hole 52 then having a shape fitting to the shape of the outer surface 8 of the skirt 7 of the tip cap 1. Tamper indicator means 54 (schematically shown on Figure 9) may be arranged between the collar 46 and rigid outer cap 50, for indicating a separation of these two parts. For example, such tamper indicator means can comprise breakable bridges or a tearable label.

When the tip cap assembly 45 is mounted on the injection device 20, the collar 46 is securely connected to the tip 27 and the outer cap 50 is engaged within the collar 46. The tip cap 1, which is securely mounted in the outer cap 50, is sealingly engaged with the tip 27, as previously described. In this embodiment, prior to the use of the injection device, the tip cap 1 is removed. To that end, the outer cap 50 - with the tip cap 1 secured inside it - is removed from the collar 46, i.e. from the injection device 20. The collar 46 remains secured to the tip 27. Therefore, the tamper indicator means, when present, are broken. Then, for example, a needle assembly 36 having a needle hub 37 provided with an outer thread 41 can be connected to the collar 46 of the injection device 20.

Reference is now made to Figures 10 to 19.

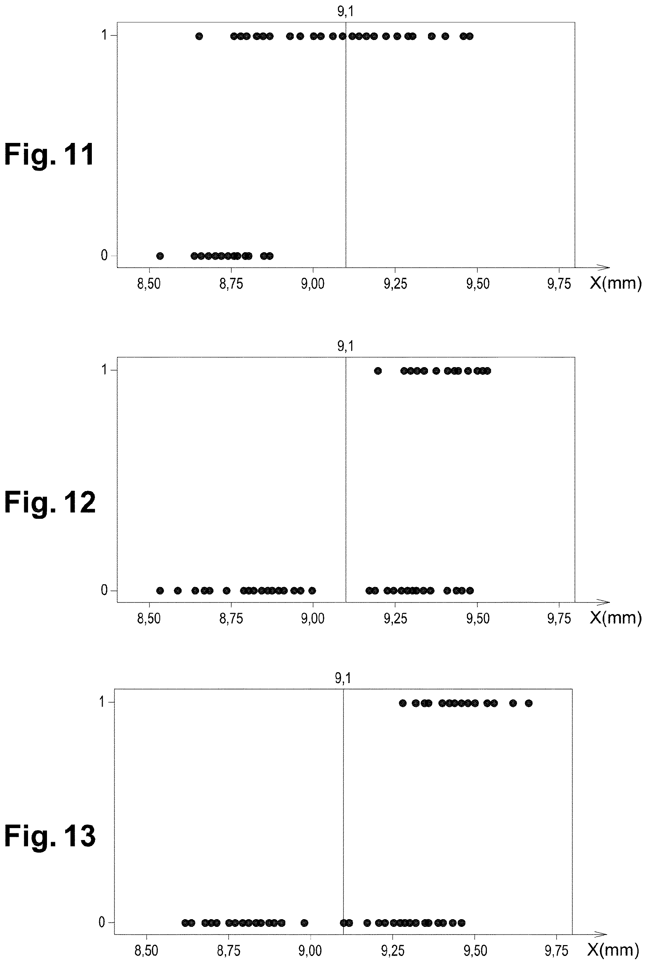

Figures 11 to 19 are graphs illustrating the improved sealing effect achieved with a tip cap according to the invention.

Scatter plots on each of these graphs show the results of leak tests performed on injection devices equipped with a tip cap of the prior art and tip caps according to the present invention.

In these graphs, the x axis corresponds to the insertion depth of the tip cap 1 onto the injection device tip 27, in millimeters (mm), when the tip cap is plugged on the tip of this injection device by a manufacturing equipment, such as a Bausch & Stroebel assembly machine.

As shown in Figure 10, the insertion depth of the tip cap 1 onto the injection device tip 27 is x = L2 - L1, where:
- L1 is the length of the injection device 20, from the proximal end 23 of the barrel 22 to the distal end 29 of the tip 27;
- and L2 is the distance between the proximal end 23 of the barrel 22 and the distal surface 4 of the tip cap 1 when the tip cap 1 is plugged on the tip 27.

This insertion depth can usually vary from 8,3 mm (where the tip cap is inserted quite deeply onto the tip) to 9,5 mm (where the tip cap is not inserted very deeply onto the tip), depending on the machine used, as well as on the type of tip caps and/or injection devices. However, in practice, this insertion depth can be adjusted more precisely when an automatic tool is used, and an insertion depth above 9,1 mm is generally not appropriate as, in such a case, the tip cap 1 is not enough inserted onto the tip 27.

According to ISO 594 standards, an injection device is qualified when no leakage occurs with a pressure inferior or equal to 3 bars applied on the plunger rod during the injection of a liquid contained into said injection device. Therefore, an extrapolation of ISO 594 standards is that a tip cap is qualified when no leakage occurs with said tip cap inserted on an injection device filled with a liquid, on which a pressure of 3 bars is applied during around 30 seconds.

Three different leak tests have been conducted to evaluate the different tip caps.

A first leak test is conducted according to an extrapolation of ISO 594 standards, as previously explained. More precisely, it consists in filing the chamber 26 of an injection device 20 closed by different tip caps with a liquid, typically water, applying a calibrated pressure of 3 bars on the plunger rod 35, and checking whether a leakage occurs or not after a predetermined period of time. In the present case, the first test has been conducted within a period of time of around 30 s. For one given tip cap 1, the test has been conducted with various insertion depths of the tip cap 1 onto the injection device tip 27.

In this first leak test, all tip caps tested as well as the barrel they are mounted onto have been sterilized. Moreover, this is to be noted that all tip caps tested as well as the barrel they are mounted onto have been submitted to different sterilizations (one sterilization cycle of Ethylene Oxyde sterilization followed by two sterilization cycles of steam sterilization).

The results of the first leak test are shown on Figures 11, 12 and 13 and show data obtained for different tip caps, respectively for a tip cap of the prior art, for a tip cap according to a first embodiment of the invention, and for a tip cap according to a second embodiment of the invention (the corresponding data of the first and second embodiments being gathered in the above table).

For one given insertion depth, the plot on the graph according to where it is located, shows whether there is a leakage or not. In other words, in Figures 11 to 13, the y axis corresponds to a "failed-unfailed" data, 0 meaning that no leakage occurred and 1 meaning that a leakage occurred.

The tip cap of the prior art has an unspecified inner cavity as compared to the tip cap of the invention which has an inner cavity having a specific design as previously described. Therefore, as it can be seen in Figure 11, a tip cap of the prior art cannot systematically prevent leakage, since, with an insertion depth below 9,1 mm, some plots are equal to the 1 value, which, as explained above, proves a leakage and therefore is not acceptable. Said tip cap of the prior art presents a design that is not satisfactorily adapted to the tip of the injection device and therefore leaks. On the contrary, the tip caps according to the first and second embodiments of the invention do pass the test.

With only such a first test, a result of "1" does not allow to conclude if the tested tip cap is a high performance tip cap - in terms of sealing level - or if it barely passed the test, meaning that one cannot ensure that leakage problems will not arise with said tip cap.

Therefore, additional tests were conducted as a complement in order to characterize the tip cap performance against leakage, for the tip cap of the prior art and for the first and second embodiments. These additional leak tests, namely a second and a third leak tests, consist in using an injection device filled with a liquid, typically water, progressively increasing the pressure applied on the plunger rod 35, and then determining the pressure at which a leakage occurs.

The second leak test was conducted with non-sterilized tip caps and injection devices. The third leak test was conducted with sterilized tip caps and barrels submitted to different sterilizations (one sterilization cycle of Ethylene Oxyde sterilization followed by two sterilization cycles of steam sterilization).

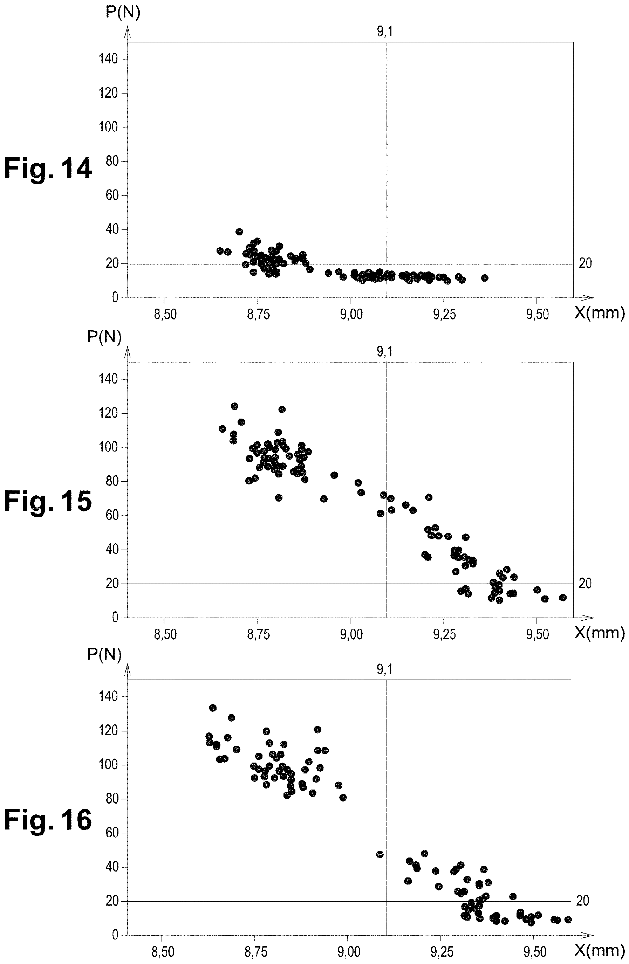

On Figures 14 to 19, which show the results of the second and third leak tests, the y axis corresponds to the pressure exerted by the plunger rod 35 on a liquid inside the chamber 26, in Newton (N), when the plunger rod 35 is moved from a proximal to a distal direction, expelling the medical fluid contained in the chamber 26. One plot on the graph corresponds to a given insertion depth of the tip cap and the corresponding limit pressure leading to a leakage. The limit drawn at 20 N on the graphs of Figures 14 to 19 corresponds to the limit acceptable for qualifying an injection device, insofar as 3 bars is a value equivalent to a 20 N force for this type of injection devices.

As a consequence, on Figures 14 to 19, a suitable tip cap shows a pressure measured and an insertion depth which are respectively higher than 3 bars (equivalent to 20 N force for this type of injection devices) and lower than 9,1 mm, as this data corresponds to the technical requirements and the usual operational conditions. In other words, a suitable tip cap should not lead to plots located in the bottom area of the graph.

The results of the second leak test are shown in Figures 14, 15 and 16, and show data obtained for different tip caps, respectively for a tip cap of the prior art, for a tip cap according to a first embodiment of the invention, and for a tip cap according to a second embodiment of the invention (the corresponding data of the first and second embodiments being gathered in the above table).

As it can be seen in Figure 14, and because of its features described above, the tip cap of the prior art cannot systematically prevent leakage, since, with an insertion depth below 9,1 mm, some plots are located under the pressure limit of 20 N, which, as explained above, is not acceptable, and confirms the results obtained for the same design during the first leak test. Said tip cap of the prior art presents a design that is not satisfactorily adapted to the tip of the injection device and therefore leaks.

On the contrary, the tip caps 1 according to the two embodiments of the present invention (as shown respectively in Figures 15 and 16) demonstrate a great improvement of the sealing effect. Indeed, both embodiments of the tip caps 1 according to the invention show that, even if the tip cap is plugged at a depth higher than 9,1 mm, an increase in the pressure above 20 N does not induce any leakage.

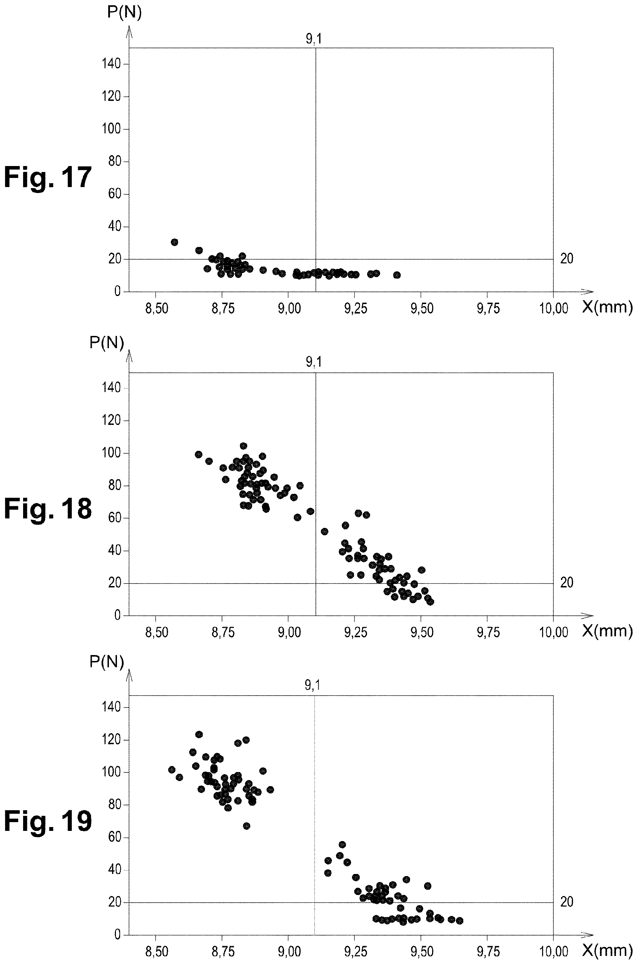

The results of the third leak test are shown in Figures 17, 18 and 19 and show data obtained for different tip caps, respectively for a tip cap of the prior art, for a tip cap according to a first embodiment of the invention, and for a tip cap according to a second embodiment of the invention (the corresponding data of the first and second embodiments being gathered in the above table).

As it can be seen in Figure 17, the tip cap of the prior art cannot systematically prevent leakage, since, with an insertion depth below 9,1 mm, some plots are located under the pressure limit of 20 N which, as explained above, is unacceptable and confirms the results obtained for the same design during the first leak test and during the second leak test.

On the contrary, as shown in Figures 18 and 19 respectively, the tip caps 1 according to both embodiments of the present invention demonstrate a great improvement of the sealing effect. Indeed, both embodiments of the tip caps 1 according to the invention show that, even if the tip cap is plugged at a depth higher than 9,1 mm, an increase in the pressure above 20 N does not induce any leakage. Finally, the results of the second and third leak tests for both embodiments of the invention are comparable, meaning the leakage performance of the tip cap 1 is independent from sterilization: even after sterilization, the specific features of the tip cap according to the invention still have very good properties in terms of sealing efficiency when plugged on an injection device such as a syringe.

Further additional tests have been conducted on the tip caps according to the possible embodiments of the invention, to ascertain that the other important properties of said tip caps are not degraded as, for example, the sterility maintenance and the ethylene oxide residue level inside the tip cap, but also the pullout force.

Indeed, it has been found that the pull out force remains in acceptable levels, that is, in a range from 4 N to 45 N. This pull out force is linked to the friction of the inner cavity 14 on the surface of the injection device tip 27 and should not be too high, in order to allow the user to remove the tip cap 1 prior to the use of the injection device 20 without difficulties.

The invention is of course not limited to the embodiments described above as examples, but is limited only by the scope of the claims.

## Claims

1. A tip cap designed to be sealingly mounted on a distally projecting tip (27) of an injection device (20) having a passageway extending therethrough, the tip cap (1) having an axis (2) and comprising:
- a closed distal end portion (3) having a substantially transverse proximal surface (5);
- a substantially cylindrical peripheral skirt (7) extending in the proximal direction from the proximal surface (5) of the distal end portion (3), said peripheral skirt (7) having an inner surface (10) designed to cooperate with the outer surface (31) of the injection device tip (27);
- a nipple (11) protruding from the proximal surface (5) of the distal end portion (3) in the proximal direction, said nipple (11) having a dome-shaped proximal portion (13) and being designed to engage the injection device tip (27);
an inner cavity (14) thus being formed in the tip cap (1), the bottom (15) of said cavity (14) being a part of the proximal surface (5) of the distal end portion (3) and having a substantially annular shape which is connected to the peripheral skirt inner surface (10) by an outer fillet (16) and to the nipple (11) by an inner fillet (17);
**characterized in that**:
- the outer fillet (16) has a radius of curvature (R16) ranging from 0,4 to 0,6 mm;
- the inner fillet (17) has a radius of curvature (R17) ranging from 0,25 to 0,35 mm;
- the nipple base width (W), substantially in the plane of the proximal surface (5) of the distal end portion (3), ranges from 1,4 to 1,6 mm;
- the dome-shaped proximal portion (13) of the nipple (11) has a radius of curvature (R13) ranging from 0,55 to 0,70 mm.

2. The tip cap according to claim 1, **characterized in that** the outer fillet (16) has a radius of curvature (R16) ranging from 0,45 to 0,55 mm, for example around 0,5 mm.

3. The tip cap according to claim 1 or claim 2, **characterized in that** the inner fillet (17) has a radius of curvature (R17) ranging from 0,27 to 0,33 mm, for example around 0,3 mm.

4. The tip cap according to any one of claims 1 to 3, **characterized in that** the nipple base width (W), substantially in the plane of the proximal surface (5) of the distal end portion (3), ranges from 1,45 to 1,55 mm, and is for example around 1,5 mm.

5. The tip cap according to any one of claims 1 to 4, **characterized in that** the dome-shaped proximal portion (13) of the nipple (11) has a radius of curvature (R13) ranging from 0,6 to 0,70 mm, for example around 0,65 mm.

6. The tip cap according to any one of claims 1 to 5, **characterized in that** the nipple axial length (L) ranges from 1,2 to 1,4 mm, and is for example around 1,3 mm.

7. The tip cap according to any one of claims 1 to 6, **characterized in that** it is made of an elastomeric material.

8. The tip cap according to any one of claims 1 to 7, **characterized in that** it is made of a material chosen among: natural rubber, acrylate-butadiene rubber, cis-polybutadiene, chloro or bromobutyl rubber, chlorinated polyethylene elastomers, polyalkylene oxide polymers, ethylene vinyl acetate, fluorosilicone rubbers, hexafluoropropylene-vinylidene fluoride-tetrafluoroethyleneterpolymers, butyl rubbers, polyisobutene, synthetic polyisoprene rubber, silicone rubbers, styrene-butadiene rubbers, tetrafluoroethylene propylene copolymers, thermoplastic-copolyesters, thermoplastic elastomers, or the like or a combination thereof.

9. A tip cap assembly for use with an injection device (20) having a passageway extending therethrough and having a distally projecting tip (27), the tip cap assembly (45) comprising:
- a collar (46) securely engageable around the injection device tip (27);
- a tip cap (1) according to any one of claims 1 to 8, sealingly engageable with the injection device tip (27);
- a rigid outer cap (50) securely disposed around the tip cap (1), and having a proximal portion engageable with the collar (46), said rigid outer cap (50) comprising tamper indicator means (54) for indicating a separation of the rigid outer cap (50) and the collar (46).

10. An injection device comprising:
- a barrel (22) having a passageway extending therethrough and having a distally projecting tip (27);
- a tip cap (1) according to any one of claims 1 to 8, the tip cap (1) being sealingly mounted on the injection device tip (27).

11. An injection device comprising:
- a barrel (22) having a passageway extending therethrough and having a distally projecting tip (27);
- a tip cap assembly (45) according to claim 9, the tip cap assembly (45) being mounted on the injection device tip (27) so that the tip cap (1) sealingly engages the injection device tip (27).

## Patentansprüche

1. Spitzenkappe, die gestaltet ist, um dichtend auf einer distal hervorspringenden Spitze (27) einer Injektionsvorrichtung (20) montiert zu werden, die einen Durchgang aufweist, der sich dort hindurch erstreckt, wobei die Spitzenkappe (1) eine Achse (2) aufweist und umfasst:
- einen geschlossenen distalen Endabschnitt (3), der eine im Wesentlichen querlaufende proximale Oberfläche (5) aufweist;
- eine im Wesentlichen zylindrische umlaufende Schürze (7), die aus der der proximalen Oberfläche (5) des distalen Endabschnitts (3) heraus sich in die proximale Richtung erstreckt, wobei die besagte umlaufende Schürze (7) eine innere Oberfläche (10) aufweist, die gestaltet ist, um mit der äußeren Oberfläche (31) der Spitze der Injektionsvorrichtung (27) zusammenzuwirken;
- einen Nippel (11) der aus der proximalen Oberfläche (5) des distalen Endabschnitts (3) in die proximale Richtung hervorspringt, wobei der besagte Nippel (11) einen kuppelförmigen proximalen Abschnitt (13) aufweist und dazu bestimmt ist, in die Spitze der Injektionsvorrichtung (27) einzugreifen;
eine innere Vertiefung (14), die somit in der Spitzenkappe (1) gebildet wird, wobei der Boden (15) der besagten Vertiefung (14) ein Teil der proximalen Oberfläche (5) des distalen Endabschnitts (3) ist und eine im Wesentlichen ringförmige Form aufweist, die durch eine äußere Auskehlung (16) mit der inneren Oberfläche der umlaufenden Schürze (10), und durch eine innere Auskehlung (17) mit dem Nippel (11) verbunden ist;
**dadurch gekennzeichnet, dass**:
- die äußere Auskehlung (16) einen Krümmungsradius (R16) in einem Bereich von 0,4 bis 0,6 mm aufweist;
- die innere Auskehlung (17) einen Krümmungsradius (R17) in einem Bereich von 0,25 bis 0,35 mm aufweist;
- die Nippel-Basisbreite (W) im Wesentlichen in der Ebene der proximalen Oberfläche (5) des distalen Endabschnitts (3) von 1,4 bis 1,6 mm reicht;
- der kuppelförmige proximale Abschnitt (13) des Nippels (11) einen Krümmungsradius (R13) in einem Bereich von 0,55 bis 0,70 mm aufweist.

2. Spitzenkappe nach Anspruch 1, **dadurch gekennzeichnet, dass** die äußere Auskehlung (16) einen Krümmungsradius (R16) in einem Bereich von 0,45 bis 0,55 mm, beispielsweise um 0,5 mm aufweist.

3. Spitzenkappe nach Anspruch 1 oder Anspruch 2, **dadurch gekennzeichnet, dass** die innere Auskehlung (17) einen Krümmungsradius (R17) in einem Bereich von 0,27 bis 0,33 mm, beispielsweise um 0,3 mm aufweist.

4. Spitzenkappe nach einem der Ansprüche 1 bis 3, **dadurch gekennzeichnet, dass** die Nippel-Basisbreite (W) im Wesentlichen in der Ebene der proximalen Oberfläche (5) des distalen Endabschnitts (3) von 1,45 bis 1,55 mm reicht, und beispielsweise um 1,5 mm ist.

5. Spitzenkappe nach einem der Ansprüche 1 bis 4, **dadurch gekennzeichnet, dass** der kuppelförmige proximale Abschnitt (13) des Nippels (11) einen Krümmungsradius (R13) in einem Bereich von 0,6 bis 0,70 mm, beispielsweise um 0,65 mm aufweist.

6. Spitzenkappe nach einem der Ansprüche 1 bis 5, **dadurch gekennzeichnet, dass** die axiale Länge des Nippels (L) von 1,2 bis 1,4 mm reicht, und beispielsweise um 1,3 mm ist.

7. Spitzenkappe nach einem der Ansprüche 1 bis 6, **dadurch gekennzeichnet, dass** sie aus einem Elastomer-Werkstoff gefertigt ist.

8. Spitzenkappe nach einem der Ansprüche 1 bis 7, **dadurch gekennzeichnet, dass** sie aus einem Werkstoff gefertigt ist, der ausgewählt wird aus: Naturkautschuk, Acrylat-Butadien-Kautschuk, Cis-Polybutadien, Chlor- oder Brombutyl-Kautschuk, chlorierte Polyethylen-Elastomere, Polyalkylenoxid-Polymere, Ethylen-Vinylacetat, Fluorsilikon-Kautschuke, Hexafluorpropylen-Vinyliden, Fluorid-Tetrafluorethyleneter-Polymere, Butylkautschuke, Polyisobuten, synthetischer Polyisopren-Kautschuk, Silikonkautschuk, Styrol-Butadien-Kautschuke, Tetrafluorethylen-Propylen-Copolymere, thermoplastische Copolyester, thermoplastische Elastomere, oder ähnliches oder eine Kombination davon.

9. Spitzenkappenanordnung zur Verwendung mit einer Injektionsvorrichtung (20), die einen Durchgang aufweist, der sich dort hindurch erstreckt, und eine sich distal erstreckende Spitze (27) aufweist, wobei die Spitzenkappenanordnung (45) umfasst:
- einen Kragen (46), der fest um die Spitze der Injektionsvorrichtung (27) einsetzbar ist;
- eine Spitzenkappe (1) nach einem der Ansprüche 1 bis 8, die dichtend in der Spitze der Injektionsvorrichtung (27) einsetzbar ist;
- eine starre äußere Kappe (50), die fest um die Spitzenkappe (1) herum angeordnet ist, und einen proximalen Abschnitt aufweist, der in den Kragen (46) einsetzbar ist, wobei die besagte starre äußere Kappe (50) Manipulationsanzeigemittel (54) zum Hinweisen auf eine Trennung der starren äußeren Kappe (50) und dem Kragen (46) umfasst.

10. Injektionsvorrichtung, umfassend:
- einen Tubus (22), der einen Durchgang aufweist, der sich dort hindurch erstreckt, und eine distal hervorspringende Spitze (27) aufweist;
- eine Spitzenkappe (1) nach einem der Ansprüche 1 bis 8, wobei die Spitzenkappe (1) dichtend auf der Spitze der Injektionsvorrichtung (27) montiert ist.

11. Injektionsvorrichtung, umfassend:
- einen Tubus (22), der einen Durchgang aufweist, der sich dort hindurch erstreckt, und ein distal hervorspringende Spitze (27) aufweist;
- eine Spitzenkappenanordnung (45) nach Anspruch 9, wobei die Spitzenkappenanordnung (45) auf der Spitze der Injektionsvorrichtung (27) montiert ist, sodass die Spitzenkappe (1) dichtend in die Spitze der Injektionsvorrichtung (27) eingreift.

## Revendications

1. Capuchon conçu pour être monté de manière étanche sur un embout (27) faisant saillie de manière distale d'un dispositif d'injection (20) ayant un passage s'étendant à travers celui-ci, le capuchon (1) ayant un axe (2) et comprenant :
- une partie d'extrémité distale fermée (3) ayant une surface proximale sensiblement transversale (5) ;
- une jupe périphérique sensiblement cylindrique (7) s'étendant dans la direction proximale depuis la surface proximale (5) de la partie d'extrémité distale (3), ladite jupe périphérique (7) ayant une surface intérieure (10) conçue pour coopérer avec la surface extérieure (31) de l'embout (27) du dispositif d'injection ;
- un téton (11) faisant saillie depuis la surface proximale (5) de la partie d'extrémité distale (3) dans la direction proximale, ledit téton (11) ayant une partie proximale en forme de dôme (13) et étant conçu pour s'engager avec l'embout (27) du dispositif d'injection ;
une cavité intérieure (14) étant ainsi formée dans le capuchon (1), le fond (15) de ladite cavité (14) étant une partie de la surface proximale (5) de la partie d'extrémité distale (3) et ayant une forme sensiblement annulaire qui est reliée à la surface intérieure de la jupe périphérique (10) par un filet extérieur (16) et au téton (11) par un filet intérieur (17) ;
**caractérisé en ce que** :
- le filet extérieur (16) a un rayon de courbure (R16) compris entre 0,4 et 0,6 mm ;
- le filet intérieur (17) a un rayon de courbure (R17) compris entre 0,25 et 0,35 mm ;
- la largeur de base (W) du téton, sensiblement dans le plan de la surface proximale (5) de la partie d'extrémité distale (3), est comprise entre 1,4 et 1,6mm;
- la partie proximale en forme de dôme (13) du téton (11) a un rayon de courbure (R13) compris entre 0,55 et 0,70 mm.

2. Capuchon selon la revendication 1, **caractérisé en ce que** le filet extérieur (16) a un rayon de courbure (R16) compris entre 0,45 et 0,55 mm, par exemple d'environ 0,5 mm.

3. Capuchon selon la revendication 1 ou 2, **caractérisé en ce que** le filet intérieur (17) a un rayon de courbure (R17) compris entre 0,27 et 0,33 mm, par exemple d'environ 0,3 mm.

4. Capuchon selon l'une quelconque des revendications 1 à 3, **caractérisé en ce que** la largeur de base (W) du téton, sensiblement dans le plan de la surface proximale (5) de la partie d'extrémité distale (3), est comprise entre 1,45 et 1,55 mm, et est par exemple d'environ 1,5 mm.

5. Capuchon selon l'une quelconque des revendications 1 à 4, **caractérisé en ce que** la partie proximale en forme de dôme (13) du téton (11) a un rayon de courbure (R13) compris entre 0,6 et 0,70 mm, par exemple d'environ 0,65 mm.

6. Capuchon selon l'une quelconque des revendications 1 à 5, **caractérisé en ce que** la longueur axiale (L) du téton est comprise entre 1,2 et 1,4 mm, et est par exemple d'environ 1,3 mm.

7. Capuchon selon l'une quelconque des revendications 1 à 6, **caractérisé en ce qu'**il est constitué d'un matériau élastomère.

8. Capuchon selon l'une quelconque des revendications 1 à 7, **caractérisé en ce qu'**il est constitué d'un matériau choisi parmi : un caoutchouc naturel, un caoutchouc acrylate-butadiène, le cis-polybutadiène, un caoutchouc chlorobutyle ou bromobutyle, des élastomères de polyéthylène chlorés, des polymères d'oxyde de polyalkylène, l'éthylène-acétate de vinyle, des caoutchoucs fluorosilicone, des terpolymères d'hexafluoropropylène-fluorure de vinylidène-tétrafluoroéthylène, des caoutchoucs butyle, le polyisobutène, un caoutchouc synthétique polyisoprène, des caoutchoucs silicone, des caoutchoucs styrène-butadiène, des copolymères de tétrafluoroéthylène-propylène, des copolyesters thermoplastiques, des élastomères thermoplastiques, ou autres analogues ou une combinaison de ceux-ci.

9. Ensemble de capuchon pour une utilisation avec un dispositif d'injection (20) ayant un passage s'étendant à travers celui-ci et ayant un embout (27) faisant saillie de manière distale, l'ensemble de capuchon (45) comprenant :
- un collier (46) pouvant être fermement engagé autour de l'embout (27) du dispositif d'injection ;
- un capuchon (1) selon l'une quelconque des revendications 1 à 8, pouvant s'engager de manière étanche avec l'embout (27) du dispositif d'injection ;
- un capuchon extérieur rigide (50) fermement disposé autour du capuchon (1), et ayant une partie proximale pouvant s'engager avec le collier (46), ledit capuchon extérieur rigide (50) comprenant un moyen d'indication d'inviolabilité (54) pour indiquer une séparation du capuchon extérieur rigide (50) et du collier (46).

10. Dispositif d'injection comprenant :
- un corps cylindrique (22) ayant un passage s'étendant à travers celui-ci et ayant un embout (27) faisant saillie de manière distale ;
- un capuchon (1) selon l'une quelconque des revendications 1 à 8, le capuchon (1) étant monté de manière étanche sur l'embout (27) du dispositif d'injection.

11. Dispositif d'injection comprenant :
- un corps cylindrique (22) ayant un passage s'étendant à travers celui-ci et ayant un embout (27) faisant saillie de manière distale ;
- un ensemble de capuchon (45) selon la revendication 9, l'ensemble de capuchon (45) étant monté sur l'embout (27) du dispositif d'injection de sorte que le capuchon (1) s'engage de manière étanche avec l'embout (27) du dispositif d'injection.
